(19)

Europäisches
Patentamt
European
Patent Office
Office européen
des brevets

(11) **EP 1 876 184 A1**

(12) **EUROPEAN PATENT APPLICATION**
published in accordance with Art. 153(4) EPC

(43) Date of publication:
**09.01.2008 Bulletin 2008/02**

(21) Application number: **06705640.8**

(22) Date of filing: **14.02.2006**

(51) Int Cl.:
*C07K 7/06* (2006.01)     *C07K 1/14* (2006.01)
*C07K 1/16* (2006.01)     *C07K 1/18* (2006.01)
*C07K 1/36* (2006.01)     *C07K 14/47* (2006.01)
*A61K 38/00* (2006.01)     *A61K 38/08* (2006.01)
*A61P 37/02* (2006.01)

(86) International application number:
**PCT/CN2006/000219**

(87) International publication number:
**WO 2006/111060 (26.10.2006 Gazette 2006/43)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **21.04.2005   CN 200510012463**

(71) Applicant: **Shi Jia Zhuang Sanlu Group Co., Ltd. Shijiazhuang 050071 (CN)**

(72) Inventors:
• **WANG, Yuliang**
  **Shijiazhuang 050071 (CN)**
• **SHENG, Qinghai**
  **Shijiazhuang 050071 (CN)**

• **FANG, Xinping**
  **Shijiazhuang 050071 (CN)**
• **XIA, Wenshui**
  **Shijiazhuang 050071 (CN)**
• **ZHAI, Hongmei**
  **Shijiazhuang 050071 (CN)**
• **WEI, Peng**
  **Shijiazhuang 050071 (CN)**

(74) Representative: **Rapisardi, Mariacristina et al Ufficio Brevetti Rapisardi Srl Via Serbelloni, 12 20122 Milano (IT)**

(54) **A METHOD FOR ISOLATING AND PURIFYING IMMUNO-MODULATING POLYPEPTIDE FROM COW PLACENTA**

(57)     The present invention provides a method for isolating and purifying immuno-modulating polypeptide from cow placenta, which is **characterized by** using the steps of anion-exchange chromatography, gel exclusion chromatography and reverse-phase high performance liquid chromatography to isolate and purify immuno-modulating polypeptide from cow placenta, identifying its activity of stimulating lymphocyte proliferation in vitro by MTT method, then determining its molecular weight by MALDI-TOF-MS, its isoelectric point by CIEF and its amino acid sequence with analyzer for protein sequencing. Since the obtained immuno-modulating polypeptide by the method according to the present invention has more than 90% purity, its bioactivity can reach medicinal standards.

**EP 1 876 184 A1**

Description

## TECHNICAL FIELD

The present invention relates to biology and medicine extraction field. More particularly, it relates to a method for isolating and purifying immuno-modulating polypeptide from cow placenta.

## BACKGROUND ART

With more people change their life to modem life style, the decline of the immunity would cause diseases. Enhancing the immunity is one of the essential approaches for resisting fatigue, improving work efficiency and preventing diseases.

The research of placenta immuno-modulating factor, the active components extracted from healthy puerperal placenta originated from China. In 1985, LIU Yuexin (Preparation and Study on Placenta Factor--A New Immunomodulator, Chinese Journal of Immunology, 1985,1(5)) disclosed extracting a small molecular active substance from healthy puerpera placenta by homogenation-dialysis for the first time, and named the active substance as placenta factor. In 1994, HUANG Chuhua et al. (Laboratory Study of Plaenta Polypeptide on Physical and ChemicalProperties and Bioactivity, PHARMACEUTICAL BIOTECHNOLOGY, 1995,2(2)) obtained placenta injection, through the following steps: washing and cuting the fresh placenta, preparing homogenate with 2 times saline, centrifugal precipitating, ultrafiltering supernatants by ultrafiltration membrane with molecular weight cut-offs of 10,000, and sterilizing and dispensing the filtrate. In 1996, ZHANG Xuerong et al. (Experimental Study on Placenta Immuno-modulating Factor, GUANGXI SCIENCES,1996,3) disclosed placenta injection was obtained by cutting off anadesma of the placenta, washing with aseptic water, cutting and weighing, preparing homogenation by high-speed tissue homogenizer with 1:1 saline, then freezing the homogenation at -20°C in refrigerator for more than 48h, defrosting in water bath at 25 °C, phacofragmentating for 15min, dealing with poly frosted-defrosted and centrifugal precipitating at low temperature, dialyzing supernatants and filtering with membrane. In 2001, XU Daidi et al. (Preparing Placenta Immuno-modulating Factor, Guangxi Medical Journal, 2001,8) prepared the placenta immuno-modulating factor refering to LIU Yuexin.

These researches show that, the main component of human placenta immuno-modulating factor is small molecular polypeptide. Large amount of animal experiments and clinic applications indicate that, placenta immuno-modulating factor is safety, nontoxic, no antigenicity, the activity would not decline for inducing antibody with long term use, and the placenta immuno-modulating factor is a safe high-efficient immunoregulator and immunostimulants. However, the present researches about placenta immuno-modulating factor mostly focus on its function and application, the reports on how to isolate and purify its main active components are rarely seen.

As a new kind of placenta resource, cow placenta, is similar with human in origin structure and components although differences between them are exit exactly. So it is important both on theory and technology that isolating and purifying immuno-modulating factor from cow placenta with different technology referring to the research on human placenta immuno-modulating factor.

## DISCLOSURE OF THE INVENTTION

## TECHNICAL PROBLEM

It is an object of the present invention to provide a method for extracting immuno-modulating polypeptide from cow placenta.

It is another object of the present invention to provide immuno-modulating polypeptide extracted from cow placenta.

## DESCRIPTION OF THE INVENTION

It is known that ion exchange chromatography is carried out in the system that employs ion exchange agent as stationary phase and liquid as mobile phase. Ion exchange agent comprises matrix, ion groups and counterion. The reaction between ion exchange agent and ion or ionic compounds in the liquid is mainly base on ion exchanging or base on absorption between ion groups of ion exchange agent and ion or ion compounds in solution. Ion exchange chromatography is carried out based on the difference of charge states (or polarities). Appropriate ion exchange agent should be chosen by dissociation properties or charge states of the objective. The conditions of absorption and elution should be controlled, main of which as ionic strength and the value of pH of eluant. Then the components in the mixture are eluted from chromatography column sequentially according to the affinity.

Gel exclusion chromatography is carried out by the movements of vertical downward and nondirectional diffusion of the molecules during them flowing through the chromatography column at a low flow rate. Macromolecules have big diameters, spread in spaces between particles instead in gel particle's small holes, and move vertically faster during elution. However, small molecules not only diffuse into spaces between gel particles but also entry gel particles inside. During the vertical movement, the small molecules diffuse from inside of the particle to spaces between the particles then entry the other particle inside. The movements of diffusion and entrance are continual. Therefore, the speed of vertical movement of small molecules is lower than that of macromolecules. As a result, macromolecules, medium molecules, small molecules are out of column sequentially, and separately successfully. The obvious advantage of gel exclusion chromatography is that the carrier it used is inert, and it is free of charge, weak absorbability, mild operate condition, wide operate temperature scope, no need of organic solvent, and has special advantage in maintaining physical and chemical properties of the compoundss.

Reversed phase chromatography is widely used to isolating. In the system, solute is retained mainly by hydrophobic interaction. Hydrophobic interaction means that the interreaction between solute molecules or between solute molecules and water molecules is far less than that between water molecules where nonpolar solute is in the aqueous solution. Therefore, solute molecules can be removed from solution easily. Different compounds has different retention time for different hydrophobic properties, and the compounds are isolated as a result.

The present inventor have done lots of research, and combined these three methods creatively, which combined the anion exchange chromatography, gel exclusion chromatography and reversed phase high performance liquid chromatography together, to extract immuno-modulating polypeptide from cow placenta, determined molecular weight and isoelectric point and sequence of the isolated and purified immuno-modulating polypeptide.

The present invention relates to immuno-modulating polypeptide isolated from cow placenta. The characteristics of the immuno-modulating polypeptide are as follows: it has a molecular weight of 2133.52Da determined by MALDI-TOF-MS (matrix assisted laser desorption ionization time of flight mass spectrometry), an isoelectric point of 3.82 determined by CIEF (Capillary isoelectric focusing), and a sequence of Tyr-X-Phe-Leu-Gly-Leu-Pro-Gly-X-Thr determined by 491-protein sequencer (Applied Biosystems, USA), wherein X is an amino acid.

The present invention also relates to a method for preparing immuno-modulating polypeptide isolated from cow placenta. The said method comprises the following steps known to those skilled in the art: washing and cutting fresh cow placenta, adding phosphate buffer of pH6.8-7.5 which 2 times (w/vol.) of placenta, preparing homogenate, centrifugating at 12000r/m, precipitating, ultrafiltering supernatants by ultrafiltration membrane with molecular weight cut-offs of 10,000, desalinating with aromatic polyamide nanofiltration membrane and freeze-dying, thus the lyophilized powder sample containing immuno-modulating polypeptide is obtained. The method also comprises dissolving the said lyophilized powder in phosphate buffer, using anion exchange chromatography, gel exclusion chromatography and reverse phase high performance liquid chromatography to isolate the solution, determining the active substance collected from reverse phase high performance liquid chromatography by RP-HPLC and capillary electrophoresis and the results both were single peak, then determining the molecular weight by MALDI-TOF-MS, isoelectric point by CIEF, and peptide sequence by protein sequencer.

According to an embodiment of the invention, the said anion exchange chromatography is performed under that dissolving 4-10:1(w:vol) the said lyophilized powder in phosphate buffer of 10-30mmol/L pH6.8-7.5 as solution sample, loading the solution sample onto anion exchange column at the flow rate of 0.5-1.5mL/min, gradient eluting at the flow rate of 0.5-1.5mL/min, wherein eluting solution A is phosphate buffer of 10-30mol/L, eluting solution B is the solution prepared by adding 0.5-1.5 mol/L NaCl solution to solution A, eluting at 0-600min solution A and 600-1000min solution B of 0-100%. Determining by MTT method through stimulating lymphocyte proliferation in vitro, screening, and the components containing immuno-modulating polypeptide is collected after separated by anion exchange chromatography.

Preferably, the ratio of the said lyophilized powder to the buffer used to dissolve the powder is 4-8:1 (w:vol.), more preferably, the ratio is 5-8:1.

Also, the buffer employed to dissolve the lyophilized powder according to the present invention is one or more solution chosen from $Na_2HPO_4$-$NaH_2PO_4$, $K_2HPO_4$-$KH_2PO_4$, $K_2HPO_4$-$NaH_2PO_4$ and/or $Na_2HPO_4$-$KH_2PO_4$ solution.

According to another embodiment of the present invention, the said lyophilized powder solution sample is 10-30mmol/L pH 6.8-7.5 $Na_2HPO_4$- $NaH_2PO_4$ buffer.

Also, the eluting solution A employed by anion exchange chromatography is one or more solution chosen from $Na_2HPO_4$-$NaH_2PO_4$, $K_2HPO_4$-$KH_2PO_4$, $K_2HPO_4$-$NaH_2PO_4$ and $Na_2HPO_4$-$KH_2PO_4$ eluting solution.

According to another embodiment of the present invention, the said gel exclusion chromatography is performed under that loading the immuno-modulating polypeptide component collected from anion exchange chromatography onto the gel exclusion column directly at the flow rate of 8-12 mL/h, the mobile phase being phosphate buffer of 10-30 mmol/L pH6.8-7.5, eluting at flow rate of 8-12mL/h with equal gradient elution, determining by MTT method through stimulating lymphocyte proliferation in vitro, screening, collecting the components containing immuno-modulating polypeptide after separated by anion exchange chromatography.

The said gel exclusion column has a length/diameter ratio of 60-90, and the volume of sample reaches to 1-10% of that of column bed.

Preferably, the said mobile phase used in gel exclusion column is one or more solutions chosen from $Na_2HPO_4$-$NaH_2PO_4$, $K_2HPO_4$-$KH_2PO_4$, $K_2HPO_4$-$NaH_2PO_4$ and $Na_2HPO_4$-$KH_2PO_4$ solution.

According to another embodiment of the present invention, the said reverse phase high performance liquid chromatography is performed under that loading the immuno-modulating polypeptide component collected from gel exclusion chromatography onto the reverse phase high performance liquid chromatography column directly at the flow rate of 0.8-1.2 mL/min, the mobile phase A being 5-10% Acetonitrile with 0.01-0.1% trifluoroacetic acid in, the mobile phase B being 40-60% Acetonitrile with 0.01-0.1% trifluoroacetic acid in, gradient eluting at flow rate of 0.8-1.2mL/h with 0-8min solution A, 8-12min solution B(0-100%), 12-16min solution B.

The said reverse phase high performance liquid chromatography column has a length/diameter ratio of 25-50, and the volume of sample reaches to 1-10% of that of column bed.

Preferably, the said mobile phase A used in the present invention is 5-8% Acetonitrile with 0.02-0.08% trifluoroacetic acid in, and mobile phase B is 40-60% Acetonitrile with 0.02-0.08% trifluoroacetic acid in.

According to another embodiment of the present invention, the said anion exchange column is equiped with anion exchange medias chosen from agarose DEAE Sepharose CL-6B, DEAE-Sepharose FF or glucan DEAE-Sephadex A-25 or DEAE-Sephadex A-50 functionalized with diethylaminoethyl.

Preferably, the said anion exchange column is equiped with DEAE Sepharose CL-6B, DEAE-Sepharose FF or DEAE-Sephadex A-25.

According to another embodiment of the present invention, the said gel exclusion chromatography column is glucan Sephadex G-25, Sephadex G-50 or Polyacrylamine gel Bio-gel-P-4, Bio-gel-P-6 or Bio-gel-P-10 with inactive porous net-like constructure, and separates substances in the protein mixture based on the sizes of molecules.

Preferably, the said gel exclusion chromatography column is a column with a media chosen from Sephadex G-25, Sephadex G-50, Bio-gel-P-4 and Bio-gel-P-6.

According to another embodiment of the present invention, the said reverse phase high performance liquid chromatography column is Sephasil peptide C18 or Polymer C18 packing with ODS (ctadecylsilyl-silica).

## ADVANTAGEOUS EFFECT

The purity of immuno-modulating polypeptide from the present invention is higher than 90%, the bioactivity of which meets the standard of medicine preparing.

The immuno-modulating polypeptide from the present invention provides plenty of stuff for preparing healthy food with immuno- modulating activity.

The industry yield of immuno-modulating polypeptide from the present invention is 1%, 1g immuno-modulating polypeptide from 100g cow placenta (dry basis based).

## DESCRIPTION OF DRAWINGS

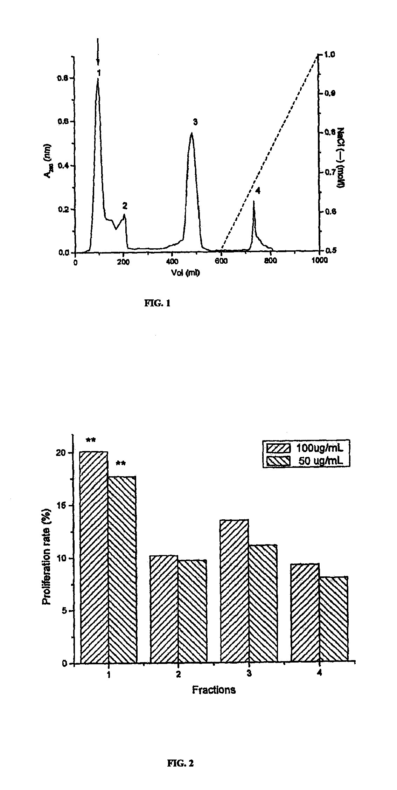

FIG. 1 is the chromatogram from the separation of immuno-modulating polypeptide in one embodiment of present invention on DEAE Sepharose CL-6B anion exchange column.

Peak 1a in FIG. 1 is the component containing immuno-modulating polypeptide.

FIG. 2 is the bar diagram of 4 components for stimulating lymphocyte proliferation in vitro, which separated from immuno-modulating polypeptide in one embodiment of present invention on DEAE Sepharose CL-6B anion exchange column..

Component 1a in FIG. 2 is the component containing immuno-modulating polypeptide.

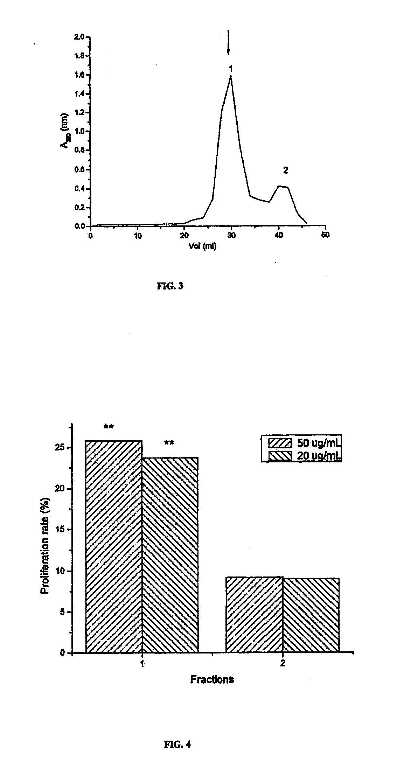

FIG. 3 is the chromatogram from the separation of immuno-modulating polypeptide in one embodiment of present invention on Sephadex G-25 gel exclusion chromatography column.

Peak 1b in FIG. 3 is the component containing immuno-modulating polypeptide.

FIG. 4 is the bar diagram of 2 components for stimulating lymphocyte proliferation in vitro, which separated from immuno-modulating polypeptide in one embodiment of present invention on Sephadex G-25 gel exclusion chromatography column.

Component 1b in FIG. 4 is the component containing immuno-modulating polypeptide.

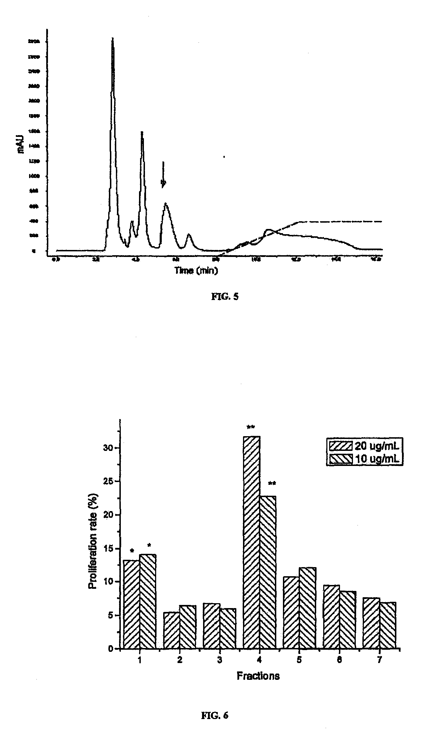

FIG. 5 is the chromatogram from the separation of immuno-modulating polypeptide in one embodiment of present invention on Sephasil peptide $C_{18}$ reversed phase high performance liquid chromatography column.

Peak 4 in FIG. 5 is the component containing immuno-modulating polypeptide.

FIG. 6 is the bar diagram of 7 components for stimulating lymphocyte proliferation in vitro, which separated from immuno-modulating polypeptide in one embodiment of present invention on Sephasil peptide $C_{18}$ reversed phase high performance liquid chromatography column.

Component 4 in FIG. 6 is the component containing immuno-modulating polypeptide.

FIG. 7 shows the purity of immuno-modulating polypeptide in one embodiment of present invention determined by Sephasil peptide $C_{18}$ reversed phase high performance liquid chromatography. The purity is higher than 90%.

FIG. 8 shows the purity of immuno-modulating polypeptide in one embodiment of present invention determined by capillary electrophoresis.

DETAILED DESCRIPTION OF THE EMBODIMENTS

In order to understand the present invention more clearly, hereinafter, preferred embodiments of the present invention will be described in detail with reference to accompanying drawings.

Detailed description of the experiment method:

1) Stimulating lymphocyte proliferation in vitro
**Culturing cells**

1 Preparing spleen lymphocyte suspension:

Remove out the mice spleen under sterile condition, separate lymphocyte by lymphocyte separation medium, regulate the cell concentration to $1 \times 10^6$/ml with complete 1640 culture solution.

2 Experiment arrangement:

Set control group and experiment group separately to identify the activity of stimulating spleen lymphocyte proliferation by every component.

3 Experiment method:

Add 100 μL mice spleen lymphocyte suspension ($1 \times 10^6$/mL) to 96 well cultured plates. Add 100 μL saline/ sample to control group and experiment group separately. Mix to homogenous gently, culture in incubator for 68h at 37°Cunder 5% $CO_2$. Add 20μl MTT to every well under sterile condition, Mix to homogenous gently, continue to culture for 4h, add 100μl dimethyl sulfoxide to every well, keep at room temperature for 10min, then determine OD of every well by ELISA at 570nm.

4 Data processing
The spleen lymphocyte proliferation rate of experiment group is calculated by the following formula:

$$Spleen\ lymphocyte\ proliferation\ rate\ (\%) = \frac{A_{test} - A_{control}}{A_{test}} \times 100\%$$

The value of P is calculated by variance analysis, and significant difference is calculate by T test.

2) Determining molecular weight of immuno-modulating polypeptide by MALDI-TOF-MS.
3) Determining isoelectric poin of immuno-modulating polypeptide by CIEF.
4) Determining sequence of immuno-modulating polypeptide by protein sequencer.
5) Determining by RP-HPLC and capillary electrophoresis.
The foresaid methods 2)-5) are the normal methods known as those skilled in the art.

**EXAMPLES**

**EXAMPLE 1**

A. PLACENTA PRECONDITIONING

Washed and cut fresh cow placenta, added phosphate buffer of pH7.0 which 2 times (w/v) of placenta, prepared homogenate, centrifugated at 12000r/m for 3 min, precipitated, ultrafiltered supernatants by ultrafiltration membrane with molecular weight cut-offs of 10,000, desalinated with aromatic polyamide nanofiltration membrane and freeze-dried, thus the lyophilized powder sample containing immuno-modulating polypeptide was obtained.

B. ANION EXCHANGE CHROMATOGRAPHY

Dissolved 30mg lyophilized powder in 5ml pH7.4 phosphate buffer with concentration of 20mmol/L $Na_2HPO_4$-$NaH_2PO_4$, loaded the solution sample onto 2.6 $\times$ 35 cm DEAE Sepharose CL-6B anion exchange column at the flow rate of 1ml/min, then gradient eluted, wherein eluting solution A was 20mmol/L $Na_2HPO_4$- $NaH_2PO_4$ phosphate buffer, eluting solution B was 20mmol/L $Na_2HPO_4$- $NaH_2PO_4$ phosphate buffer with 1mol/L NaCl solution added in, and eluted at 0-600min solution A and 600-1000min solution B of 0-100%.(0-100% meant from 100% solution A and 0% solution B to 0% solution A and 100% solution B)

The anion exchange chromatogram showed in Fig. 1. The activity determined by stimulating lymphocyte proliferation in vitro showed in Fig. 2. The activity determining result showed that the composition from anion exchange chromatography contained immuno-modulating polypeptide (the peak that arrow indicated).

C. GEL EXCLUSION CHROMATOGRAPHY

Loaded foresaid immuno-modulating polypeptide component 1a collected from anion exchange chromatography onto 1.0 $\times$ 75 cm Sephadex G-25 gel exclusion column with the flow rate of 8 mL/h. The mobile phase was 20mmol/L pH7.4 $Na_2HPO_4$- $NaH_2PO_4$ phosphate buffer. Equal gradient eluted.

The gel exclusion chromatogram showed in Fig. 3. The activity determined by stimulating lymphocyte proliferation in vitro showed in Fig. 4. The activity determining result showed that the composition from gel exclusion chromatography contained immuno-modulating polypeptide (the peak that arrow indicated).

D. REVERSE PHASE HIGH PERFORMANCE LIQUID CHROMATOGRAPHY

Loaded foresaid immuno-modulating polypeptide component 1b collected from gel exclusion chromatogray onto Sephasil peptide $C_{18}$ reverse phase high performance liquid chromatography column with the flow rate of 1 mL/min. The mobile phase A was 10% Acetonitrile with 0.05% trifluoroacetic acid in, the mobile phase B was 60% Acetonitrile with 0.05% trifluoroacetic acid in, gradient eluted with 0-8min solution A, 8-12min solution B(0-100%), then 12-16min solution B.

The reverse phase high performance liquid chromatogram showed in Fig. 5. The activity determined by stimulating lymphocyte proliferation in vitro showed in Fig. 6. The activity determining result showed that the componebt 4 was high-purity immuno-modulating polypeptide.

E. IDENTIFYING AND ANALYZING

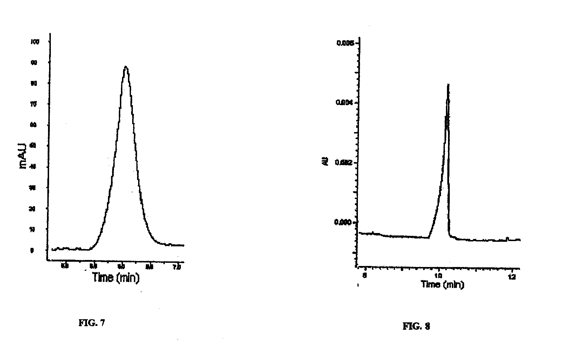

Desalinated component 4 with nanofiltration membrane and freeze-dried to obtain immuno-modulating polypeptide product. Identified the product purity by Sephasil peptide $C_{18}$ RP-HPLC and capillary electrophoresis. The results showed in Fig. 7 and Fig. 8 separately, and both were single peak.

The molecular weight of immno-modulating polypeptide determined by MALDI-TOF-MS was 2133.52 Da.

The isoelectric poin of immuno-modulating polypeptide determined by CIEF was 3.82.

The peptide sequence of immuno-modulating polypeptide determined by 491-protein sequencer was Tyr-X-Phe-Leu-Gly-Leu-Pro-Gly-X-Thr (X, an amino acid)o

**EXAMPLE 2**

A. PLACENTA PRECONDITIONING

Washed and cut fresh cow placenta, added phosphate buffer of pH7.0 which 2 times (w/v) of placenta, prepared homogenate, centrifugated at 12000r/m, precipitated, ultrafiltered supernatants by ultrafiltration membrane with molecular weight cut-offs of 10,000, desalinated with aromatic polyamide nanofiltration membrane and freeze-dried, thus the lyophilized powder sample containing immuno-modulating polypeptide was obtained.

## B. ANION EXCHANGE CHROMATOGRAPHY

Dissolved 30mg lyophilized powder in 5ml pH6.8 phosphate buffer with concentration of 20mmol/L $Na_2HPO_4$-$NaH_2PO_4$, loaded the solution sample on $2.6 \times 35$ cm DEAE Sepharose CL-6B anion exchange column at the flow rate of 1ml/min, then gradient eluted, wherein eluting solution A was 20mmol/L $Na_2HPO_4$- $NaH_2PO_4$ phosphate buffer, eluting solution B was 20mmol/L $Na_2HPO_4$- $NaH_2PO_4$ phosphate buffer with 1mol/L NaCl solution added in, and eluted at 0-600min solution A and 600-1000min solution B of 0-100%.

The anion exchange chromatogram and the activity determined by stimulating lymphocyte proliferation in vitro were similar with Fig.1 and Fig. 2 separately. The activity determining result showed that the composition from anion exchange chromatography contained immuno-modulating polypeptide 1(the peak that arrow indicated).

## C. GEL EXCLUSION CHROMATOGRAPHY

Loaded foresaid immuno-modulating polypeptide component 1a collected from anion exchange chromatography onto $1.0 \times 75$ cm Sephadex G-25 gel exclusion column with the flow rate of 10 mL/h. The mobile phase was 20mmol/L pH6.8 $Na_2HPO_4$- $NaH_2PO_4$ phosphate buffer. Equal gradient eluted.

The gel exclusion chromatogram and the activity determined by stimulating lymphocyte proliferation in vitro were similar with Fig.3 and Fig. 4 separately. The activity determining result showed that the composition from gel exclusion chromatography contained immuno-modulating polypeptide (the peak that arrow indicated).

## D. REVERSE PHASE HIGH PERFORMANCE LIQUID CHROMATOGRAPHY

Loaded foresaid immuno-modulating polypeptide component 1b collected from gel exclusion chromatogray onto Sephasil peptide $C_{18}$ reverse phase high performance liquid chromatography column with the flow rate of 1 mL/min. The mobile phase A was 8% Acetonitrile with 0.05% trifluoroacetic acid in, the mobile phase B was 55% Acetonitrile with 0.05% trifluoroacetic acid in, gradient eluted with 0-8min solution A, 8-12min solution B(0-100%), then 12-16min solution B.

The reverse phase high performance liquid chromatogram and the activity determined by stimulating lymphocyte proliferation in vitro were similar with Fig.5 and Fig. 6 separately. The activity determining result showed that the componebt 4 was high-purity immuno-modulating polypeptide.

## E. IDENTIFYING AND ANALYZING

Desalinated component 4 with nanofiltration membrane and freeze-dried to obtain immuno-modulating polypeptide product. Identified the product purity by Sephasil peptide $C_{18}$ RP-HPLC and capillary electrophoresis. The results were similar with Fig. 7 and Fig. 8 separately, and both were single peak.

The molecular weight of immno-modulating polypeptide determined by MALDI-TOF-MS was 2133.52 Da.

The isoelectric poin of immuno-modulating polypeptide determined by CIEF was 3.82.

The peptide sequence of immuno-modulating polypeptide determined by 491-protein sequencer was Tyr-X-Phe-Leu-Gly-Leu-Pro-Gly-X-Thr (X, an amino acid)。

## EXAMPLE 3

### A. PLACENTA PRECONDITIONING

Washed and cut fresh cow placenta, added phosphate buffer of pH7.0 which 2 times (w/v) of placenta, prepared homogenate, centrifugated at 12000r/m, precipitated, ultrafiltered supernatants by ultrafiltration membrane with molecular weight cut-offs of 10,000, desalinated with aromatic polyamide nanofiltration membrane and freeze-dried, thus the lyophilized powder sample containing immuno-modulating polypeptide was obtained.

### B. ANION EXCHANGE CHROMATOGRAPHY

Dissolved 30mg lyophilized powder in 5ml pH7.2 phosphate buffer with concentration of 20mmol/L $Na_2HPO_4$-$NaH_2PO_4$, loaded the solution sample onto $2.6 \times 35$ cm DEAE Sepharose CL-6B anion exchange column at the flow rate of 1ml/min, then gradient eluted, wherein eluting solution A was 20mmol/L $Na_2HPO_4$- $NaH_2PO_4$ phosphate buffer, eluting solution B was 20mmol/L $Na_2HPO_4$- $NaH_2PO_4$ phosphate buffer with 1mol/L NaCl solution added in, and eluted at 0-600min solution A and 600-1000min solution B of 0-100%.

The anion exchange chromatogram and the activity determined by stimulating lymphocyte proliferation in vitro were similar with Fig.1 and Fig. 2 separately. The activity determining result showed that the composition from anion exchange chromatography contained immuno-modulating polypeptide 1(the peak that arrow indicated).

## C. GEL EXCLUSION CHROMATOGRAPHY

Loaded foresaid immuno-modulating polypeptide component 1a collected from anion exchange chromatography onto 1.0 × 75 cm Sephadex G-25 gel exclusion column with the flow rate of 12 mL/h. The mobile phase was 20mmol/L pH7.2 $Na_2HPO_4$- $NaH_2PO_4$ phosphate buffer. Equal gradient eluted.

The gel exclusion chromatogram and the activity determined by stimulating lymphocyte proliferation in vitro were similar with Fig.3 and Fig. 4 separately. The activity determining result showed that the composition from gel exclusion chromatography contained immuno-modulating polypeptide (the peak that arrow indicated).

## D. REVERSE PHASE HIGH PERFORMANCE LIQUID CHROMATOGRAPHY

Loaded foresaid immuno-modulating polypeptide component 1b collected from gel exclusion chromatogray onto Sephasil peptide $C_{18}$ reverse phase high performance liquid chromatography column with the flow rate of 1 mL/min. The mobile phase A was 5% Acetonitrile with 0.05% trifluoroacetic acid in, the mobile phase B was 45% Acetonitrile with 0.05% trifluoroacetic acid in, gradient eluted with 0-8min solution A, 8-12min solution B(0-100%), then 12-16min solution B.

The reverse phase high performance liquid chromatogram and the activity determined by stimulating lymphocyte proliferation in vitro were similar with Fig.5 and Fig. 6 separately. The activity determining result showed that the componebt 4 was high-purity immuno-modulating polypeptide.

## E. IDENTIFYING AND ANALYZING

Desalinated component 4 with nanofiltration membrane and freeze-dried to obtain immuno-modulating polypeptide product. Identified the product purity by Sephasil peptide $C_{18}$ RP-HPLC and capillary electrophoresis. The results were similar with Fig. 7 and Fig. 8 separately, and both were single peak.

The molecular weight of immno-modulating polypeptide determined by MALDI-TOF-MS was 2133.52 Da.

The isoelectric poin of immuno-modulating polypeptide determined by CIEF was 3.82.

The peptide sequence of immuno-modulating polypeptide determined by 491-protein sequencer was Tyr-X-Phe-Leu-Gly-Leu-Pro-Gly-X-Thr (X, an amino acid).

## EXAMPLE 4

A. PLACENTA PRECONDITIONING

Washed and cut fresh cow placenta, added phosphate buffer of pH7.0 which 2 times (w/v) of placenta, prepared homogenate, centrifugated at 12000r/m, precipitated, ultrafiltered supernatants by ultrafiltration membrane with molecular weight cut-offs of 10,000, desalinated with aromatic polyamide nanofiltration membrane and freeze-dried, thus the lyophilized powder sample containing immuno-modulating polypeptide was obtained.

B. ANION EXCHANGE CHROMATOGRAPHY

Dissolved 30mg lyophilized powder in 5ml pH7.2 phosphate buffer with concentration of 20mmol/L $Na_2HPO_4$-$NaH_2PO_4$, loaded the solution sample on 2.6 × 35 cm DEAE Sepharose CL-6B anion exchange column at the flow rate of 1ml/min, then gradient eluted, wherein eluting solution A was 20mmol/L $Na_2HPO_4$- $NaH_2PO_4$ phosphate buffer, eluting solution B was 20mmol/L $Na_2HPO_4$- $NaH_2PO_4$ phosphate buffer with 1mol/L NaCl solution added in, and eluted at 0-600min solution A and 600-1000min solution B of 0-100%.

The anion exchange chromatogram and the activity determined by stimulating lymphocyte proliferation in vitro were similar with Fig.1 and Fig. 2 separately. The activity determining result showed that the composition from anion exchange chromatography contained immuno-modulating polypeptide 1(the peak that arrow indicated).

C. GEL EXCLUSION CHROMATOGRAPHY

Loaded foresaid immuno-modulating polypeptide component 1a collected from anion exchange chromatography onto 1.0 × 75 cm Sephadex G-25 gel exclusion column with the flow rate of 12 mL/h. The mobile phase was 20mmol/L pH7.2 $Na_2HPO_4$- $NaH_2PO_4$ phosphate buffer. Equal gradient eluted.

The gel exclusion chromatogram and the activity determined by stimulating lymphocyte proliferation in vitro were similar with Fig.3 and Fig. 4 separately. The activity determining result showed that the composition from gel exclusion chromatography contained immuno-modulating polypeptide (the peak that arrow indicated).

D. REVERSE PHASE HIGH PERFORMANCE LIQUID CHROMATOGRAPHY

Loaded foresaid immuno-modulating polypeptide component 1b collected from gel exclusion chromatogray onto Sephasil peptide $C_{18}$ reverse phase high performance liquid chromatography column with the flow rate of 1 mL/min. The mobile phase A was 5% Acetonitrile with 0.05% trifluoroacetic acid in, the mobile phase B was 60% Acetonitrile with 0.05% trifluoroacetic acid in, gradient eluted with 0-8min solution A, 8-12min solution B(0-100%), then 12-16min solution B.

The reverse phase high performance liquid chromatogram and the activity determined by stimulating lymphocyte proliferation in vitro were similar with Fig.5 and Fig. 6 separately. The activity determining result showed that the componebt 4 was high-purity immuno-modulating polypeptide.

E. IDENTIFYING AND ANALYZING

Desalinated component 4 with nanofiltration membrane and freeze-dried to obtain immuno-modulating polypeptide product. Identified the product purity by Sephasil peptide $C_{18}$ RP-HPLC and capillary electrophoresis. The results were similar with Fig. 7 and Fig. 8 separately, and both were single peak.

The molecular weight of immno-modulating polypeptide determined by MALDI-TOF-MS was 2133.52 Da.

The isoelectric poin of immuno-modulating polypeptide determined by CIEF was 3.82.

The peptide sequence of immuno-modulating polypeptide determined by 491-protein sequencer was Tyr-X-Phe-Leu-Gly-Leu-Pro-Gly-X-Thr (X-an amino acid)o

**EXAMPLE 5**

A. PLACENTA PRECONDITIONING

Washed and cut fresh cow placenta, added phosphate buffer of pH7.0 which 2 times (w/v) of placenta, prepared homogenate, centrifugated at 12000r/m, precipitated, ultrafiltered supernatants by ultrafiltration membrane with molecular weight cut-offs of 10,000, desalinated with aromatic polyamide nanofiltration membrane and freeze-dried, thus the lyophilized powder sample containing immuno-modulating polypeptide was obtained.

B. ANION EXCHANGE CHROMATOGRAPHY

Dissolved 30mg lyophilized powder in 5ml pH7.4 phosphate buffer with concentration of 20mmol/L $Na_2HPO_4$-$NaH_2PO_4$, loaded the solution sample onto 2.6 × 35 cm DEAE Sepharose CL-6B anion exchange column at the flow rate of 1ml/min, then gradient eluted, wherein eluting solution A was 20mmol/L $Na_2HPO_4$- $NaH_2PO_4$ phosphate buffer, eluting solution B was 20mmol/L $Na_2HPO_4$- $NaH_2PO_4$ phosphate buffer with 1mol/L NaCl solution added in, and eluted at 0-600min solution A and 600-1000min solution B of 0-100%.(0-100% meant from 100% solution A and 0% solution B to 0% solution A and 100% solution B)

The anion exchange chromatogram and the activity determined by stimulating lymphocyte proliferation in vitro were similar with Fig.1 and Fig. 2 separately. The activity determining result showed that the composition from anion exchange chromatography contained immuno-modulating polypeptide 1(the peak that arrow indicated).

C. GEL EXCLUSION CHROMATOGRAPHY

Loaded foresaid immuno-modulating polypeptide component 1a collected from anion exchange chromatography onto 1.0 × 75 cm Sephadex G-25 gel exclusion column with the flow rate of 10 mL/h. The mobile phase was 20mmol/L pH7.4 $Na_2HPO_4$- $NaH_2PO_4$ phosphate buffer. Equal gradient eluted.

The gel exclusion chromatogram and the activity determined by stimulating lymphocyte proliferation in vitro were similar with Fig.3 and Fig. 4 separately. The activity determining result showed that the composition from gel exclusion chromatography contained immuno-modulating polypeptide (the peak that arrow indicated).

## D. REVERSE PHASE HIGH PERFORMANCE LIQUID CHROMATOGRAPHY

Loaded foresaid immuno-modulating polypeptide component 1b collected from gel exclusion chromatogray onto Sephasil peptide $C_{18}$ reverse phase high performance liquid chromatography column with the flow rate of 1 mL/min. The mobile phase A was 10% Acetonitrile with 0.05% trifluoroacetic acid in, the mobile phase B was 40% Acetonitrile with 0.05% trifluoroacetic acid in, gradient eluted with 0-8min solution A, 8-12min solution B(0-100%), then 12-16min solution B.

The reverse phase high performance liquid chromatogram was similar with fig. 5, and the activity determined by stimulating lymphocyte proliferation in vitro was similar with Fig. 6. The activity determining result showed that the componebt 4 was high-purity immuno-modulating polypeptide.

## E. IDENTIFYING AND ANALYZING

Desalinated component 4 with nanofiltration membrane and freeze-dried to obtain immuno-modulating polypeptide product. Identified the product purity by Sephasil peptide $C_{18}$ RP-HPLC and capillary electrophoresis. The results were similar with Fig. 7 and Fig. 8 separately, and both were single peak.

The molecular weight of immno-modulating polypeptide determined by MALDI-TOF-MS was 2133.52 Da.

The isoelectric point of immuno-modulating polypeptide determined by CIEF was 3.82.

The peptide sequence of immuno-modulating polypeptide determined by 491-protein sequencer was Tyr-X-Phe-Leu-Gly-Leu-Pro-Gly-X-Thr (X, an amino acid)$_o$

The solution B used by reverse phase high performance liquid chromatography was 40% Acetonitrile with 0.05% trifluoroacetic acid in; other operate conditions were as the same as example 1.

The foresaid embodiments are used to explain the method for separating immuno-modulating polypeptide from cow placenta, do not limit the scope of the present invention. However, it will be apparent to those skilled in the art that various modifications and variations can be made for carrying out the same purposes of the present invention, therein without departing from the spirit and scope of the invention.

## Claims

1.    An immuno-modulating polypeptide isolated from cow placenta, **characterized in that** the molecular weight of the immuno-modulating polypeptide is 2133.52Da, isoelectric poin is 3.82, and peptide sequence is Tyr-X-Phe-Leu-Gly-Leu-Pro-Gly-X-Thr, wherein X is an amino acid.

2.    A method for preparing immuno-modulating polypeptide isolated from cow placenta comprising: removing out fresh cow placenta, watering, cutting, homogenating, centrifugating, ultrafiltering, desalinating with nanofiltration membrane and freeze-dried, **characterized in** tha the method also comprises: dissolving lyophilized powder obtained by the said freeze-drying step in phosphate buffer, using anion exchange chromatography, gel exclusion chromatography and reverse phase high performance liquid chromatography to isolate the solution, desalinating the component collected from reverse phase high performance liquid chromatography with nanofiltration membrane and freeze-drying, determining the immuno-modulating polypeptide product by RP-HPLC and capillary electrophoresis and the results both were single peak, then determining the molecular weight by MALDI-TOF-MS, isoelectric point by CIEF, and peptide sequence by protein sequencer.

3.    The method according to claim 2, **characterized in that** the said anion exchange chromatography is performed under that dissolving 4-10:1(w:vol) the said lyophilized powder in phosphate buffer of 10-30mmol/L pH6.8-7.5 as solution sample, loading the solution sample onto anion exchange column at the flow rate of 0.5-1.5mL/min, gradient eluting at the flow rate of 0.5-1.5mL/min, wherein eluting solution A is phosphate buffer of 10-30mol/L, eluting solution B is the solution prepared by adding 0.5-1.5 mol/L NaCl solution to solution A, eluting at 0-600min solution A and 600-1000min solution B of 0-100%. Determining by MTT method through stimulating lymphocyte proliferation in vitro, screening, and the components containing immuno-modulating polypeptide is collected after separated by anion exchange chromatography.

4.    The method according to claim 2, **characterized in that** the said gel exclusion chromatography is performed under that loading the immuno-modulating polypeptide component collected from anion exchange chromatography onto the gel exclusion column directly at the flow rate of 8-12 mL/h, the mobile phase being phosphate buffer of 10-30 mmol/L pH6.8-7.5, eluting at flow rate of 8-12mL/h with equal gradient elution, determining by MTT method through stimulating lymphocyte proliferation in vitro, screening, collecting the components containing immuno-modulating polypeptide after separated by anion exchange chromatography.

5. The method according to claim 2, **characterized in that** the said reverse phase high performance liquid chromatography is performed under that loading the immuno-modulating polypeptide component collected from gel exclusion chromatography onto the reverse phase high performance liquid chromatography column directly at the flow rate of 0.8-1.2 mL/min, the mobile phase A being 5-10% Acetonitrile with 0.01-0.1% trifluoroacetic acid in, the mobile phase B being 40-60% Acetonitrile with 0.01-0.1% trifluoroacetic acid in, gradient eluting at flow rate of 0.8-1.2mL/h with 0-8min solution A, 8-12min solution B(0-100%), 12-16min solution B.

6. The method according to claim 3, **characterized in that** the said anion exchange column is equiped with anion exchange medias chosen from agarose DEAE Sepharose CL-6B,or DEAE-Sepharose FF or glucan DEAE-Sephadex A-25 or DEAE-Sephadex A-50 functionalized with diethylaminoethyl.

7. The method according to claim 4, **characterized in that** the said gel exclusion chromatography column is glucan Sephadex G-25, Sephadex G-50 or Polyacrylamine gel Bio-gel-P-4, Bio-gel-P-6 or Bio-gel-P-10 with inactive porous net-like constructure, and separates substances in the protein mixture based on the sizes of molecules.

8. The method according to claim 5, **characterized in that** the said reverse phase high performance liquid chromatography column is Sephasil peptide $C_{18}$ or Polymer $C_{18}$ packing with ODS reverse phase packing.

9. The method according to claim 3, **characterized in** tha the said solution sample is 10-30mmol/L pH 6.8-7.5$Na_2HPO_4$-$NaH_2PO_4$ buffer.

FIG. 1

FIG. 2

FIG. 3

FIG. 4

**FIG. 5**

**FIG. 6**

FIG. 7

FIG. 8

# INTERNATIONAL SEARCH REPORT

| International application No. |
|---|
| PCT/CN2006/000219 |

**A. CLASSIFICATION OF SUBJECT MATTER**

See extra sheet

According to International Patent Classification (IPC) or to both national classification and IPC

**B. FIELDS SEARCHED**

Minimum documentation searched (classification system followed by classification symbols)

$IPC^8$  C07K, A61K38, A61P37

Documentation searched other than minimum documentation to the extent that such documents are included in the fields searched

Electronic data base consulted during the international search (name of data base and, where practicable, search terms used)

WPI, EPODOC, PAJ, CPRS, CNKI, CA, MEDLINE, cow, placenta, extract+, purify,

polypeptide?, chromatography, HPLC, immuno+, regulate+, MTT, DEAE

**C. DOCUMENTS CONSIDERED TO BE RELEVANT**

| Category* | Citation of document, with indication, where appropriate, of the relevant passages | Relevant to claim No. |
|---|---|---|
| Y | Journal of Wuxi University of Light Industry, Vol.24, No.1, Jan.2005 (01.2005), SHENG Q. et al. "Research on the beef placenta extraction and stimulation of fibroblast proliferation activity", page 39-page 40 | 2-9 |
| Y | Acta Academiae Medicinae Qingdao Universitatis, Vol.31, No.4, 1995, LANG G. et al. "Purification of hepatocyte growth factor from cow placenta", page 273-page 276 | 2-9 |
| Y | Chinese Pharmacological Bulletin, Vol.20, No.4, Apr.2004 (04.2004), HUANG D. et al. "The purification and identification of anticoagulation protein from human placenta", page 468-page 471 | 2-9 |

☒ Further documents are listed in the continuation of Box C.     ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim (S) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 22.Mar.2006(22.03.2006) | 0 6 · APR 2006 (0 6 · 0 4 · 2 0 0 6) |
| Name and mailing address of the ISA/CN<br>The State Intellectual Property Office, the P.R.China<br>6 Xitucheng Rd., Jimen Bridge, Haidian District, Beijing, China<br>100088<br>Facsimile No. 86-10-62019451 | Authorized officer<br><br>WANG, Boli<br><br>Telephone No. 86-10-62085225 |

Form PCT/ISA /210 (second sheet) (April 2005)

| | INTERNATIONAL SEARCH REPORT | International application No. |
| --- | --- | --- |
| | | PCT/CN2006/000219 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | Journal of Chromatography B, Vol.755, No.1&2, 2001, ERIKSSON H. et al. "Characterization of human placental alkaline phosphatase by activity and protein assays, capillary electrophoresis and matrix-assisted laser desorption/ionization time-of-flight mass spectrometry", page 311-page 319 | 2-9 |
| A | RU, C1, 2007420 (UNIV MED) 15.Feb.1994(15.02.1994), abstract and examples 1-3 of the description | 1-9 |
| A | JP, A, 6116286 (KOWA CO LTD et al.) 26.Apr.1994(26.04.1994), abstract and claim 1 | 1 |
| A | JP, A, 4020289 (MOCHIDA PHARM CO LTD) 23.Jan.1992(23.01.1992), claim 1-3 and examples 1-6 of the description | 1-9 |

Form PCT/ISA /210 (continuation of second sheet ) (April 2005)

## INTERNATIONAL SEARCH REPORT

International application No.

PCT/CN2006/000219

CLASSIFICATION OF SUBJECT MATTER

C07K 7/06 (2006.01) i

C07K 1/14 (2006.01) i

C07K 1/16 (2006.01) i

C07K 1/18 (2006.01) i

C07K 1/36 (2006.01) i

C07K14/47(2006.01) i

A61K38/00(2006.01) i

A61K38/08(2006.01) i

A61P37/02(2006.01) i

Form PCT/ISA /210 (extra sheet) (April 2005)

# INTERNATIONAL SEARCH REPORT

| | |
|---|---|
| International application No. | |
| PCT/CN2006/000219 | |

**Box No. II    Observations where certain claims were found unsearchable (Continuation of item 2 of first sheet)**

This international search report has not been established in respect of certain claims under Article 17(2)(a) for the following reasons:

1. ☐  Claims Nos.:
    because they relate to subject matter not required to be searched by this Authority, namely:

2. ☐  Claims Nos.:
    because they relate to parts of the international application that do not comply with the prescribed requirements to such an extent that no meaningful international search can be carried out, specifically:

3. ☐  Claims Nos.:
    because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

Group I, claim 1, drawn to an immuno-modulating polypeptide from cow placenta.
Group II, claims 2-9, drawn to a preparative method of immuno-modulating polypeptide isolated from cow placenta.

1. ☐  As all required additional search fees were timely paid by the applicant, this international search report covers all searchable claims.

2. ☒  As all searchable claims could be searched without effort justifying an additional fees, this Authority did not invite payment of any additional fee.

3. ☐  As only some of the required additional search fees were timely paid by the applicant, this international search report covers only those claims for which fees were paid, specifically claims Nos.:

4. ☐  No required additional search fees were timely paid by the applicant. Consequently, this international search report is restricted to the invention first mentioned in the claims; it is covered by claims Nos.:

**Remark on protest**         ☐  The additional search fees were accompanied by the applicant's protest and, where applicable, the payment of a protest fee.

☐  The additional search fees were accompanied by the applicant's protest but the applicable protest fee was not paid within the time limit specified in the invitation.

☐  No protest accompanied the payment of additional search fees.

Form PCT/ISA /210 (continuation of first sheet (2)) (April 2005)

## INTERNATIONAL SEARCH REPORT
Information on patent family members

| International application No. |
| --- |
| PCT/CN2006/000219 |

| Patent Documents referred in the Report | Publication Date | Patent Family | Publication Date |
| --- | --- | --- | --- |
| RU, C1, 2007420 | 15.Feb.1994(15.02.1994) | NONE | |
| JP, A, 6116286 | 26.Apr.1994(26.04.1994) | NONE | |
| JP, A, 4020289 | 23.Jan.1992(23.01.1992) | NONE | |

Form PCT/ISA /210 (patent family annex) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LIU YUEXIN.** Preparation and Study on Placenta Factor--A New Immunomodulator. *Chinese Journal of Immunology,* 1985, vol. 1 (5
- **HUANG CHUHUA et al.** Laboratory Study of Plaenta Polypeptide on Physical and ChemicalProperties and Bioactivity. *PHARMACEUTICAL BIOTECHNOLO-GY,* 1994, vol. 2 (2
- **ZHANG XUERONG et al.** Experimental Study on Placenta Immuno-modulating Factor. *GUANGXI SCIENCES,* 1996, vol. 3
- **XU DAIDI et al.** Preparing Placenta Immuno-modulating Factor. *Guangxi Medical Journal,* 2001, vol. 8